# EUROPEAN PATENT APPLICATION

(11) **EP 2 292 635 A1**
(43) Date of publication of application: **09.03.2011**
(21) Application number: 10011937.9
(22) Date of filing: 17.06.2004
(51) Int. Cl.: C07K 1/04

(54) **Improved process for supported phase synthesis**

(30) Priority: 04.07.2003 EP 03015188
(62) Divisional of application: 04739972.0
(71) Applicant: Lonza Ltd., 4052 Basel (CH)
(72) Inventor: Cool, Vincent, 7850 Marcq (BE); Forni, Luciano, 7100 La Louvière (BE); Monnaie, Didier, 7110 Houdang Aimeries (BE); Ronvaux, Alain, 7900 Leuze-en-Hainaut (BE)
(74) Representative: Weber, Joachim

(57) **Abstract**

The present invention relates to an improved process for supported phase synthesis, in particular for supported phase peptide synthesis, that makes use of salts, in particular of quaternary ammonium salts.

## Description

The present invention concerns an improved process for supported phase synthesis.

In solid phase synthesis (SPS), also called supported phase synthesis, an oligomer is assembled on a support by attaching sequentially appropriately protected monomers or other oligomers. This is done by sequential steps of coupling and deprotection, eventually separated by washing cycles.

The monomer is a building block of the molecule to be assembled on the support. For example, but not restricted to, the monomer is an appropriately protected amino acid, an appropriately protected nucleotide, an appropriately protected peptide nucleic acid, an appropriately protected saccharide or any closely modified structure of the same family.

The oligomer is a structure made by assembling at least 2 or more of the monomers from a same family, or a combination thereof. The definition of oligomer comprises bio-oligomers, which includes but is not restricted to, peptides, oligonucleotides (DNA, RNA), oligosaccharides, peptide nucleic acids (PNA) or a combination thereof. The oligomer may be linear or branched.

The support is a product to which the oligomer or first monomer is attached. This allows a thorough washing, to be performed when the oligomer attached to the support is in the solid state. Examples of supports are polystyrene beads, zeolites, controlled pore glass beads, cellulose, PEG resins, or polyamide, without being restricted thereto. Another example of a useful support is a polymer that may exist in a soluble state and can be precipitated by addition of an appropriate solvent in order to allow a thorough washing to take place.

The support on which the solid phase synthesis is performed is functionalized by a linker.

A preferred example of a support is a polystyrene - 1% divinylbenzene resin which is often used in solid phase peptide synthesis.

The term attaching or attachment means generally the formation of a covalent bond and is used for covalent bonds between any of the following: it may be the covalent bond between the first monomer and the support, wherein this bond is formed via a linker, or the bond formed between any of the monomers in the oligomer. The bond between the first monomer and the support, which is formed via a linker, and the bond between monomers in the oligomer, may make use of any functionality of the monomer, e.g. functional groups in its backbone or side chain. In solid phase peptide synthesis, the first amino acid may be attached to the support via benzylic type linkers. The linker is an appropriate molecular structure that is covalently attached to the support, and that will release the target oligomer under adequate chemical conditions. Benzylic type linkers such as benzyl ester or monoalkyloxybenzyl ester are often used, however, other linkers can also be considered. Benzylic type linkers release the target oligomer under acidolytic conditions.

Under thorough washing is understood any technical method that will eliminate the residual by-products or reagents from the previous step, e.g. coupling or deprotection reagents. A thorough washing may be performed in a batch mode or in a continuous mode process. Examples of a continuous mode process are the thorough washing of the support with a solvent in a column or the elimination of by-products or reagents through physical means like centrifugation.

In solid phase peptide synthesis (SPPS) the peptide is assembled on a polystyrene bead as support. Construction of a peptide chain on a support instead of synthesis in solution has obvious benefits: separation of the intermediate peptides from soluble reagents and solvents can be effected simply by filtration and washing with consequent savings in time and labour over the corresponding operations in solution synthesis.

The general principles of solid phase peptide synthesis are as follows (see Fmoc Solid Phase Peptide Synthesis, A Practical Approach, by W.C. Chan and P.D. White, Oxford University Press, 2000, p. 41-76; Solid Phase Synthesis, A Practical Guide, by Steven A. Kates and Fernando Albericio, Marcel Dekker Inc., 2000, p. 275-330; Solid-Phase Peptide Synthesis, by Gregg B. Fields, Academic Press, 1997, pages 3-83): generally, the C-terminal residue of the target peptide is attached to a support. The side chains of the amino acids can also be used for attachment to the linker. Most of, or preferably all of the functional groups in amino acid side chains must be masked with permanent protecting groups that are not affected by the reaction conditions employed during peptide chain assembly. The α-amino group of each amino acid to couple, is temporarily protected. After initial attachment of the first amino acid, the temporary protecting group masking the α-amino group is removed. Thereafter, washings are performed. Then, an excess of the second amino acid is introduced, with the carboxy group of this amino acid being activated for amide bond formation by reaction with a coupling reagent. After this coupling, excess reagents are removed by washing. Then, the protecting group is removed from the N-terminus of the dipeptide. Another thorough washing takes place prior to the addition of the third amino acid residue. This process is repeated until the desired peptide sequence is assembled. In a final step, the peptide is released from the support.

Extensive washings are required after each coupling step and after each cleavage of an α-amino protecting group, i.e. after each N-deprotection step, in order to eliminate deleterious chemical entities that will otherwise lead to sequence errors in the final peptide. In particular, if an activated amino acid is still present during the N-deprotection step because the washing after the coupling was not done properly, unwanted coupling will take place with this amino acid and lead to impurities corresponding to double addition of the same amino acid in the peptide sequence. Similarly, in some cases, if the washing after the N-deprotection and before addition of a new amino acid is not done properly, cleavage reagents will remain in the solution and lead to double amino acid addition during the next coupling step.

To avoid these double addition sequences in the classical approaches, about 6 to 10 washings are necessary after each coupling and another 6 to 10 washings are necessary after each N-deprotection step. This means that for each amino acid about 12 to 20 washings are necessary in a classical approach, each of them using about 10 ml solvent / gr dry unloaded support (see Peptide Synthesis Protocols, by Michael W. Pennington and Ben M. Dunn, vol. 35, Humana Press, Chapter 1, Paragraph 3.2.1; and Fmoc Solid Phase Peptide Synthesis, A Practical Approach, by W.C. Chan and P.D. White, Oxford University Press, 2000, Table 2 on page 15 in combination with p. 43).
Reduction of the number of washing steps and the amount of solvent necessary in each individual washing step would be an important progress in terms of saving solvent and time.

This applies not only to solid phase peptide synthesis but analogously to any synthesis process wherein an oligomer is assembled on a support.

The problem to be solved by the present invention was therefore to provide an improved process which greatly reduces the total amount of solvent and the process time, compared to the state of the art process.

It has now been found that addition of salts during supported phase synthesis significantly improves the wash efficiency. It is even possible to perform the process without performing any washings between the coupling and the deprotection steps.

In one aspect, the invention therefore provides a process for attaching an appropriately protected monomer or oligomer to another monomer or oligomer which is protected by a protecting group and which is attached to a support, comprising the following steps:
a) cleave the protecting group from the monomer or oligomer attached to the support; and then
b) perform a thorough washing; and then
c) add an appropriately protected monomer or oligomer and couple it to the monomer or oligomer that is attached to the support, to form a covalent bond;
characterized in that during the process, a salt (Xⁿ⁺)m(Y^{m-})ₙ which is soluble in a solvent used in this process , is added, wherein, if the salt (Xⁿ⁺)m(Y^{m-})ₙ is added in step c), the solvent is neither a chloroform/phenol nor a chloroform/trifluoroethanol mixture.

In another aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support; and then
b) perform a thorough washing; and then
c) add an α-amino protected amino acid having an unprotected C-terminus and couple it to the amino acid or peptide that is attached to the support and that now has an unprotected N-terminus, to form an amide bond;characterized in that during the process a salt (Xⁿ⁺)m(Y^{m-})ₙ, which is soluble in a solvent used in this process , is added, wherein, if the salt (Xⁿ⁺)m(Y^{m-})ₙ is added in step c), the solvent is neither a chloroform/phenol nor a chloroform/trifluoroethanol mixture.

Preferably, the processes according to the invention additionally comprise the following step:
d) perform a thorough washing; wherein step d) is performed after step c).

In a preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step a) a salt (Xⁿ⁺)m(Y^{m-})ₙ, which is soluble in a solvent used in this step, is added.

Step a) is known as deprotection step and step c) is known as coupling step in solid phase peptide synthesis.

It is important that the amino acid or peptide that is added in step c) according to the invention is protected at its amino terminus but has an unprotected C-terminus.

The term peptide as used in this invention encompasses peptides of any length, including short oligopeptides such as dipeptides or tripeptides. In (Xⁿ⁺)m(Y^{m-})ₙ, X represents a cation, n represents the charge of the cation, Y represents an anion and m represents the charge of the anion.

Any salt (Xⁿ⁺)m(Y^{m-})ₙ that has at least 0.01 % by weight solubility in the solvent may be used. Xⁿ⁺ may be any cation, but is preferably ammonium, phosphonium or sulfonium. Y^{m-} may be any anion but is preferably fluoride, chloride, bromide, iodide, hydroxide, carbonate or hydrogenocarbonate. Salts in which Y^{m-} is selected from nitrate, phosphate, hydrogenophosphate, dihydrogenophosphate, tetrafluoroborate, hexafluorophosphate, acetate, carboxylates, cyanides, isocyanates, tetra-alkylborate, tetrarylborate, trifluoroacetate, tosylate or mesylate may be used. Preferably, the salt (Xⁿ⁺)m(Y^{m-})ₙ, is selected from the group of quaternary ammonium salts, ionic liquids, phosphonium salts, inorganic cation or sulfonium salts. The present invention also encompasses any mixture of these salts.

A particularly improved washing efficiency is achieved when the quaternary ammonium salt is selected from benzyltrimethylammonium hydroxide (BTMA hydroxide), benzyltrimethylammonium chloride (BTMA chloride), benzyltrimethylammonium carbonate (BTMA carbonate); the phosphonium salt is tetrabutylphosphonium bromide; and the sulfonium salt is triethylsulfonium tetrafluoroborate. Any mixture of these salts is also encompassed by this invention.

The salt (Xⁿ⁺)m(Y^{m-})ₙ is typically used in a concentration of 1 to 2 weight % by volume solvent in the thorough washing. The concentration may be decreased to 0.1 weight % or increased to its solubility limit in the solvent used.

By addition of the salt (Xⁿ⁺)m(Y^{m-})ₙ also in the thorough washing after cleavage of the α-amino protecting group, or in the step of coupling an α-amino protected amino acid to the peptide on the support or in the thorough washing after this coupling, the wash efficiency can be even further improved. It is therefore preferred to add the salt (Xⁿ⁺)m(Y^{m-})n not only in step a but additionally in any one or more of the other steps b), c) or d).

In another preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step b) a salt (Xⁿ⁺)m(Y^{m-})ₙ, which is soluble in a solvent used in this step, is added.

It is preferred to add the salt (Xⁿ⁺)m(Y^{m-})ₙ not only in step b), but additionally in any one or more of the other steps a), c) or d).

In another preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step c) a salt (Xⁿ⁺)m(Y^{m-})ₙ, which is soluble in a solvent used in this step, is added, wherein the solvent is neither a chloroform/phenol nor a chloroform/trifluoroethanol mixture.

It is preferred to add the salt (Xⁿ⁺)m(Y^{m-})ₙ not only in step c), but additionally in any one or more of the other steps a), b) or d).

In another preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step d) a salt (Xⁿ⁺)m(Y^{m-})ₙ, which is soluble in a solvent used in this step, is added.

It is preferred to add the salt (Xⁿ⁺)m(Y^{m-})ₙ not only in step d), but additionally in any one or more of the other steps a), b) or c).

A wide variety of solvents may be chosen for all steps of the process. Preferably, the solvent is selected from DMF (N,N-dimethylformamide), NMP (N-methylpyrrolidinone), DCM (dichloromethane), ACN (acetonitrile), esters of acetic acid, acetone, dioxane, glymes ethers, low alkyl carbonates, DMSO (dimethylsulphoxide) , DME (dimethoxy ethyl ether), DMEU (1,3-dimethyl-2-imidazolidinone), DMPU (1,3-dimethyl-3,4,5,6-tetrahydro-2(1H)-pyrimidinone), 1,2-dichloroethane, isopropanol, tert-butanol, methylethylketone, formamide, sulpholane, CHC1₃, CC1₄, THF (tetrahydrofuran), DMA (N,N-dimethylacetamide), supercritical fluids, ionic liquids, water or any mixture of theses solvents. Most preferably the solvent is N,N-dimethylformamide, N-methylpyrrolidinone or a mixture of them.

According to the invention, the thorough washing b) that takes place after the cleavage of the α-amino protecting group from the peptide preferably comprises not more than 8, most preferably not more than 5 washing steps. This is a considerable improvement over the prior art solid phase peptide synthesis process where about 10 washings are necessary in order to remove the cleavage reagents properly and to avoid double addition sequences.

The thorough washing d) according to the invention, which may take place after the coupling of an α-amino protected amino acid, preferably comprises not more than 5 washing steps and most preferably not more than 2 washing steps, or it can be skipped completely. This is again a considerable improvement over the prior art solid phase peptide synthesis process where 6 to 10 washings are necessary in order to properly remove excess amino acid and to avoid double addition sequences.

The volume of solvent used in each single wash step according to the invention is about 1 to 9 ml/gr dry support, compared to about 10 ml solvent / gr dry unloaded support in the prior art approach.

Dry support means the support in its dry state before any solvent has been added to it.

Generally, each washing step lasts usually for about 2 minutes and can be reduced to about 1 minute each. The individual washing time can be increased up to 60 minutes and more. The thorough washing can also be done in a continuous flow mode instead of individual washing steps, in that case, the flow rate must be adapted. The process as described above may be performed at a temperature between 0 to 50°C, preferably between 0 to 35°C and most preferably between 18 to 22°C. The process is usually performed under atmospheric pressure but it is also applicable under higher or lower pressure.

In the coupling step c) any well known coupling agent such as DIC (1,3-diisopropylcarbodiimide), DCC (1,3-dicyclohexylcarbodiimide), EDC (N-ethyl, N'- (3-dimethyl-aminopropyl)carbodiimide hydrochloride), PyBOP (Benzotriazol-1-yloxytripyrrolidinophosphonium hexafluorophosphate) or HBTU (O-benzotriazol-1-yl-N,N,N',N'-tetramethyluronium tetrafluoroborate) may be added. According to this invention, it is preferable to use DIC.

Suitable α-amino protecting groups for solid phase peptide synthesis are well known in the art (see Fmoc Solid Phase Peptide Synthesis, A Practical Approach, by W.C. Chan and P.D. White, Oxford University Press, 2000; Solid Phase Synthesis, A Practical Guide, by Steven A. Kates and Fernando Albericio, Marcel Dekker Inc., 2000; Solid-Phase Peptide Synthesis, by Gregg B. Fields, Academic Press, 1997).
One group of known α-amino protecting groups is cleavable under basic conditions. This group encompasses Fmoc (9-fluorenylmethoxycarbonyl) and Nsc (p-Nitrophenylsulphonylethoxycarbonate) but is not limited thereto. The preferred cleavage reagent for this group of α-amino protecting groups is piperidine but other bases such as morpholine may also be used. Optionally, it is possible to add DBU (1,8-diazabicyclo[5.4.0]undec-7 ene) as an additionnal cleavage reagent.

When base-cleavable α-amino protecting groups as defined above are used and the washing is not properly done, the cleavage reagents such as piperidine will remain in residual amounts in the solvent during the next coupling step. Thereby, premature cleavage of α-amino protecting groups during the coupling step takes place which again may result in double addition sequences. It has been found that addition of an acid in the thorough washing b) as defined above helps to protonate residual cleavage reagents such as piperidine, and enhance the removal efficiency of these cleavage reagents.
Any acid that is inert to the linker used on the support may be added in cycle b). It is preferably selected from HOBt (1-hydroxybenzotriazole), HOSu (N-hydroxysuccinamide), TFA (trifluoroacetic acid), HOOBt (3-hydroxy-1,2,3-benzotriazin-4(3H)-one), HOAt (7-aza-1-hydroxybenzotriazole), HCl, H₂SO₄, HNO₃, H₃PO₄. Most preferably, the acid is HOBt. If the thorough washing b) consists of 5 washing steps, it is preferred to add the acid in the second or third step.

Another group of α-amino protecting groups is cleavable under acid conditions. This group encompasses Boc (tert-butoxycarbonyl), Trt (Trityl) and Bpoc (2-p-Biphenylisopropyloxycarbonyl) but is not limited thereto. The preferred cleavage reagents, added in step a), for this group of α-amino protecting groups is TFA (trifluoroacetic acid) in DCM (dichloromethane), followed by a treatment with a base, such as TEA (triethylamine). The base is added in step b).

Another group of α-amino protecting groups considered by this invention is stable under acidic and basic conditions. Examples of such a protecting group are Benzyloxycarbonyl and Allyloxycarbonyl which may be cleaved respectively by catalytic hydrogenation or by palladium based chemistry.

All the above-mentioned embodiments concern the process of coupling a single amino acid or peptide to a another amino acid or peptide linked to a support. This process may be repeated in order to add several amino acids or peptides and to assemble a longer peptide.

Hence, this invention also relates to a process for synthesising a peptide comprising:
a') attaching a first amino acid or peptide, having an α-amino protecting group, via its C-terminus on a functionalized support;
b') perform the process as described above with the following amino acid or peptide foreseen in the sequence;
c') repeat step b') with the appropriate amino acids or peptides until the desired sequence is achieved; and
d') cleave the assembled peptide from the support by an appropriate method.

This invention also relates to the use of a salt (Xⁿ⁺)m(Y^{m-})ₙ in solid phase peptide synthesis for improving the washing of the peptide resin.

More specifically, it relates to the use of a salt (Xⁿ⁺)m(Y^{m-})ₙ in solid phase peptide synthesis for improving the elimination of excess amino acids or cleavage regents.

The process applicability is not scale dependent; it can for example be performed for a few milligrams or at the scale of several tons.

The following examples are provided for illustrative purposes.

### Example 1

In this comparative example, no salt (Xⁿ⁺)m(Y^{m-})ₙ was used in any of the step.

A hexapeptide with the sequence Fmoc-Gln(Trt)-Asp(OtBu)-Ile-Met-Ser(tBu)-Arg(Pbf)-OH, wherein Trt (trityl), OtBu (tert-butylester), tBu (tert-butylether), Pbf (2,2,4,6,7-Pentamethyldihydro-benzofuran-5-sulfonyl) are side chain protecting groups, was synthesised on a 2-chlorotrityl polystyrene resin, according to the following protocol. The solvent volume in liter in each wash step was 4 times the weight in kilogram of the starting Fmoc-Arg(Pbf) loaded dry resin.
1) Swell and wash 5.33 g of the resin loaded with Fmoc-Arg(Pbf)-OH
2) Cleave the α-amino protecting group Fmoc by adding 3 times during 10 minutes a NMP solution comprising 1 volume % DBU and 5 volume % piperidine.
3) Wash once with NMP during 2 minutes
4) Wash once with 5 weight % HOBt in NMP during 2 minutes
5) Wash 7 times with NMP during 2 minutes
6) Add 1.409 g Fmoc-Ser(tBu)-OH and couple it to the resin-linked Arg(Pbf) in NMP, using 0.76ml DIC, 0.25 g HOBt. Kaiser monitoring is performed
7) Wash 9 times with NMP, during 2 minutes each
8) Treat 3 times during 10 minutes with 5 volume % piperidine / 1 volume % DBU in NMP, in order to cleave the Fmoc protecting group
9) Wash once with NMP during 2 minutes
10) Wash once with 5 weight % HOBt in NMP during 2 minutes
11) Wash 7 times with NMP during 2 minutes
12) Repeat steps 6-11 with the next amino acids Fmoc-Met-OH (1.43 g), Fmoc-Ile-OH (1.28 g), Fmoc-Asp(OtBu)-OH (1.535 g), Fmoc-Gln(Trt)-OH (1.93 g)
13) Cleave the hexapeptide from the resin

Kaiser monitoring is a rapid visual and semi-quantitative test which allows to evaluate the completeness of the coupling reaction in solid phase peptide synthesis (see Kaiser E et al., Anal. Biochemistry, USA, 1970 34 n° 2, p. 595-598).

### Example 2

In this example according to the invention, BTMA chloride salt (40% w/w solution in MeOH) was used in the Fmoc protecting group cleavage (step a).

The same hexapeptide as in Example 1 with the sequence Fmoc-Gln(Trt)-Asp(OtBu)-Ile-Met-Ser(tBu)-Arg(Pbf)-OH, wherein Trt, OtBu, tBu, Pbf are side chain protecting groups, was synthesised on an identical 2-chlorotrityl polystyrene resin (same lot number) as used in example 1, according to the following protocol.

The solvent volume in liter in each wash step was 4 times the weight in kilogram of the starting Fmoc-Arg(Pbf) loaded dry resin.
1) Swell and wash 5.09 g of the resin loaded with Fmoc-Arg(Pbf)-OH
2) Cleave the α-amino protecting group Fmoc by adding 3 times during 10 minutes a NMP solution comprising 1 volume % DBU, 5 volume % piperidine and 2 weight % BTMA chloride
3) Wash once with NMP, during 2 minutes
4) Wash once with 5 weight % HOBt in NMP during 2 minutes
5) Wash 3 times with NMP during 2 minutes
6) Add 1.48 g Fmoc-Ser(tBu)-OH and couple it to the resin-linked Arg(Pbf) in NMP, using 0.72 ml DIC, 0.24 g HOBt. Kaiser monitoring is performed
7) Wash 2 times with NMP, during 2 minutes each
8) Treat 3 times during 10 minutes with 5 volume % piperidine / 1 volume % DBU and 2 weight % BTMA chloride in NMP, in order to cleave the Fmoc protecting group
9) Wash once with NMP, during 2 minutes
10) Wash once with 5 weight % HOBt in NMP during 2 minutes
11) Wash 3 times with NMP during 2 minutes
12) Repeat steps 6-11 with the next amino acids Fmoc-Met-OH (1.44 g), Fmoc-Ile-OH (1.30 g), Fmoc-Asp(OtBu)-OH (1.57 g), Fmoc-Gln(Trt)-OH (2.14 g)
13) Cleave the hexapeptide from the resin

### Results

Net yield and HPLC purity were analysed for the final hexapeptide, as produced according to example 1 and 2.

| | Example 1 (without BTMA chloride) | Example 2 (with BTMA chloride) |
|---|---|---|
| Washes after coupling | 9 | 2 |
| Washes after Fmoc cleavage | 9 | 5 |
| HPLC purity | 74.7 % | 93.9 % |
| Net yield (based powder rate | 67.6 % | 74.5 % |
| and peptide content by aminoacid analysis) | | |

These results clearly show that addition of a salt as defined according to this invention in the cleavage step of the α-amino protecting group gives higher yield and higher peptide purity, hence less sequence errors. This improvement is reached with only 7 wash steps in total per each aminoacid incorporation, compared to 18 in the comparative example.

### Example 3

In this comparative example, no salt (Xⁿ⁺)m(Y^{m-})ₙ was used in any of the step.

A heptapeptide with the sequence Fmoc- Ser(tBu)-Tyr(tBu)-Arg(Pbf)-Lys(Boc)-Val-Leu-Gly-OH, wherein tBu (tert-butylether), Pbf (2,2,4,6,7-Pentamethyldihydrobenzofuran-5-sulfonyl) are side chain protecting groups, was synthesised on a 2-chlorotrityl polystyrene resin, according to the following protocol.

The solvent volume in liter in each wash step was 3.5 times the weight in kilogram of the starting Fmoc-Gly loaded dry resin.
1) Swell and wash 4.15 g the starting resin loaded with Fmoc-Gly-OH
2) Cleave the α-amino protecting group Fmoc by adding 3 times during 10 minutes a NMP solution comprising 1 volume % DBU and 5 volume % piperidine.
3) Wash once with NMP during 2 minutes
4) Wash once with 5 weight % HOBt in NMP during 2 minutes
5) Wash 8 times with NMP during 2 minutes
6) Add 1.98 g Fmoc-Leu-OH and couple it to the resin-linked Gly in NMP, using 1.05 ml DIC, 0.331 g HOBt. Kaiser monitoring is performed
7) Wash 9 times with NMP, during 2 minutes each
8) Treat 3 times during 10 minutes with 5 volume % piperidine / 1 volume % DBU in NMP, in order to cleave the Fmoc protecting group
9) Wash once with NMP during 2 minutes
10) Wash once with 5 weight % HOBt in NMP during 2 minutes
11) Wash 4 times with NMP during 2 minutes
12) Repeat steps 6-11 with the next amino acids Fmoc-Val-OH (1.91 g), Fmoc-Lys(Boc)-OH (2.43 g), Fmoc-Arg(Pbf)-OH (332 g), Fmoc-Tyr(tBu)-OH (2.28 g), Fmoc-Ser(tBu)-OH (1.84 g).
13) Cleave the peptide from the resin

### Example 4

In this example according to the invention, BTMA hydroxide salt was used in the washing d) after coupling.

The same heptapeptide as in Example 3 with the sequence Fmoc-Ser(tBu)-Tyr(tBu)-Arg(Pbfl-Lys(Boc)-Val-Leu-Gly-OH, wherein tBu (tert-butylether), Pbf (2,2,4,6,7-Pentamethyldihydro-benzofuran-5-sulfonyl) are side chain protecting groups, was synthesised on an identical 2-chlorotrityl polystyrene resin (same lot number) as used in Example 3, according to the following protocol.

The solvent volume in liter in each wash step was 4 times the weight in kilogram of the starting Fmoc-Gly loaded dry resin.
1) Swell and wash 4.67 g of the resin, load Fmoc-Gly-OH
2) Cleave the α-amino protecting group Fmoc by adding 3 times during 10 minutes a NMP solution comprising 1 volume % DBU and 5 volume % piperidine.
3) Wash once with NMP during 2 minutes
4) Wash once by 5 weight % HOBt in NMP during 2 minutes
5) Wash 3 times by NMP during 2 minutes
6) Add 2.23 g Fmoc-Leu-OH and couple it to the resin-linked Gly in NMP, using 1.18 ml DIC, 0.386 g HOBt. Kaiser monitoring is performed
7) Wash once with NMP, during 2 minutes and once during 10 minutes with NMP containing 2 weight % BTMA hydroxide
8) Treat 3 times during 10 minutes with 5 volume % piperidine / 1 volume % DBU in NMP, in order to cleave Fmoc
9) Wash once with NMP during 2 minutes
10) Wash once with 5 weight % HOBt in NMP during 2 minutes
11) Wash 3 times with NMP during 2 minutes
12) Repeat steps 6-11 with the next amino acids Fmoc-Val-OH (2.14 g), Fmoc-Lys(Boc)-OH (2.96 g), Fmoc-Arg(Pbf)-OH (4.09 g), Fmoc-Tyr(tBu)-OH (2.90 g), Fmoc-Ser(tBu)-OH (2.42 g).
13) Cleave the peptide from the resin

### Results

HPLC purity was analysed for most of the fragments during the synthesis of the peptide, as produced according to example 3 and 4.

| | Example 3 (without BTMA hydroxide) | Example 4 (with BTMA hydroxide) |
|---|---|---|
| Washes after coupling | 9 | 2 |
| Washes after Fmoc cleavage | 6 | 5 |
| Total amount of wash solvent | 52.5 ml | 28 ml |
| per gr dry starting resin for adding one amino acid | | |
| HPLC purity of fragments: | | |
| Fmoc-Leu-Gly-OH | 94.9 % | 95.0 % |
| Fmoc-Val-Leu-Gly-OH | 90.4 % | 94.0 % |
| Fmoc-Arg(Pbf)-Lys(Boc)Val-Leu-Gly-OH | 90.0 % | 91.0 % |
| Fmoc-Ser(tBu)-Tyr(tBu)- | 88.2 % | 91.7 % |
| Arg(Pbf)-Lys(Boc)Val-Leu-Gly-OH | | |

The HPLC results clearly show that addition of a salt as defined according to this invention in the washing after the coupling reaction gives comparable or higher purity. This result is achieved by only 7 wash steps and 28 ml solvent in total compared to 15 wash steps and 52.5 ml solvent in the comparative example.

## Claims

1. A process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
**characterized in that** at least in step a) or c), a salt (Xⁿ⁺)m(Y^{m-})ₙ, which is soluble in a solvent used in this step, is added, wherein the solvent is neither a chloroform/phenol nor a chloroform/trifluoroethanol mixture, wherein X represents a cation, n represents the charge of the cation, Y represents an anion and m represents the charge of the anion, and wherein said salt is selected from the group of quaternary ammonium salts, ionic liquids, phosphonium salts, sulfonium salts, inorganic salts or any mixture thereof.

2. The process according to claim 1, wherein (Y^{m-})ₙ is selected from the group of fluoride, chloride, bromide, iodide, hydroxide, carbonate, hydrogenocarbonate, nitrate, phosphate, hydrogenophosphate, dihydrogenophosphate, tetrafluoroborate, hexafluorophosphate, acetate, carboxylates, cyanides, isocyanates, tetraalkylborates, tetra-arylborates, trifluoroacetate, tosylate, mesylate or any mixture thereof, wherein Y represents an anion, m represents the charge of the anion and n represents the charge of the cation.

3. The process according to claim 1 or 2, wherein the quaternary ammonium salt is selected from benzyltrimethylammonium hydroxide, benzyltrimethylammonium chloride or benzyltrimethylammonium carbonate, the phosphonium salt is tetrabutylphosphonium bromide and the sulfonium salt is triethylsulfonium tetrafluoroborate.

4. The process according to any of claims 1 to 3, wherein the salt added in step a is also added in one or more of the other steps.

5. The process according to any of claims 1 to 4, wherein the α-amino protecting group is Fmoc (9-fluorenylmethoxycarbonyl) or Nsc (p-Nitrophenylsulphonylethoxycarbonate) or any other base-cleavable protecting group.

6. The process according to any of claims 1 to 4, wherein the α-amino protecting group is Boc (tert-butoxycarbonyl), Trt (trityl), Bpoc (2-p-Biphenylisopropyloxycarbonyl) or any other acid-cleavable protecting group.

7. The process according to any of claims 1 to 4, wherein the α-amino protecting group is selected so that neither acid nor base treatment is required for its cleavage.

8. A process for synthesising a peptide comprising:
a') attaching a first amino acid or peptide, having an α-amino protecting group, via its C-terminus to a functionalized support;
b') perform the process according to any of claims 1 to 7 with the following amino acid or peptide foreseen in the sequence;
c') repeat step b' with the appropriate amino acids or peptides until the desired sequence is achieved; and
d') cleave the assembled peptide from the support by an appropriate method.

9. Use of a salt (Xⁿ⁺)m(Y^{m-})ₙ in the process of claim 1 for improving the washing of the peptide resin, wherein X represents a cation, n represents the charge of the cation, Y represents an anion and m represents the charge of the anion, and wherein said salt is defined as in claim 1.

10. Use according to claim 9 for improving the elimination of excess amino acids or cleavage reagents.


SEQUENCE LISTING

<110>  Lonza AG

<120>  Improved process for supported phase synthesis

<130>  LP2086

<160>  3

<170>  PatentIn version 3.3

<210>  1
<211>  6
<212>  PRT
<213>  Artificial

<220>
<223>  Synthetic construct

<400>  1

Gln Asp Ile Met Ser Arg
1               5

<210>  2
<211>  7
<212>  PRT
<213>  Artificial

<220>
<223>  Synthetic construct

<400>  2

Ser Tyr Arg Lys Val Leu Gly
1               5

<210>  3
<211>  5
<212>  PRT
<213>  Artificial

<220>
<223>  Synthetic construct

<400>  3

Arg Lys Val Leu Gly
1               5